# FASCICULE DE BREVET EUROPEEN

(11) **EP 0 923 914 B1**
(45) Date de publication et mention de la délivrance du brevet: **19.03.2003**
(21) Numéro de dépôt: 97403053.8
(22) Date de dépôt: 16.12.1997
(51) Int. Cl.: A61F 2/16

(54) **Dispositif de pliage et de maintien plié d'un implant intraoculaire souple**
Vorrichtung zum Falten und Halten eines weichen intraokularen Implantates
Device for folding and holding a soft intraocular implant

(43) Date de publication de la demande: 23.06.1999
(73) Titulaire: Moria SA, 92160 Antony (FR)
(72) Inventeur: Duprat, Alain, 31120 Roquettes (FR)
(74) Mandataire: Robert, Jean-Pierre

(56) Documents cités:
- EP-A- 0 590 201
- US-A- 5 100 410
- US-A- 5 290 293
- US-A- 5 292 324

## Description

La présente invention concerne un dispositif de préparation juste avant son insertion dans l'oeil, d'un implant souple formant notamment cristallin artificiel.

On utilise de plus en plus les implants souples lors de l'opération de la cataracte et la mise en place d'un cristallin artificiel, ces implants présentant l'avantage d'une incision de petite taille pour leur introduction dans l'oeil. Une fois introduits, ces implants se déplient élastiquement pour retrouver leur forme initiale de lentille optique à l'intérieur de l'oeil. Pour faciliter le travail du chirurgien, il est utile de préparer à l'avance l'implant et de le maintenir dans sa forme pliée afin que le chirurgien n'ait qu'à le saisir au moyen d'une pince adaptée pour l'introduire dans l'oeil.

Un tel dispositif est par exemple décrit dans le document EP-A-590 201.

Par la présente invention on propose un dispositif destiné à assurer de manière plus simple cette fonction, c'est-à-dire à exécuter le pliage de l'implant et à le maintenir plié à la disposition du chirurgien.

A cet effet, l'invention a donc pour objet un dispositif de pliage et de maintien plié d'un implant intraoculaire souple qui comporte deux mâchoires, chaque mâchoire ayant une surface active en forme de dièdre rentrant et étant montée coulissante par rapport à l'autre le long d'une direction parallèle à l'un des côtés du dièdre rentrant entre une première position où elles sont en contact l'une de l'autre par l'extrémité du côté parallèle à la direction de coulissement et au moins une seconde position dans laquelle elles sont éloignées l'une de l'autre d'une distance au moins égale au diamètre de l'implant à plier, tandis qu'au moins un organe élastique est attelé entre les mâchoires dont l'effet tend à les maintenir au contact l'une de l'autre, chaque mâchoire étant par ailleurs solidaire d'un poussoir situé en regard de sa surface active et au-delà de l'autre mâchoire.

Ce dispositif se comporte comme une pince normalement fermée qui, lorsque l'opérateur l'actionne, s'ouvre pour dégager un espace entre les mâchoires permettant d'accueillir un implant et, lorsque l'opérateur cesse de l'actionner, se referme sur l'implant en le forçant à former un pli. Dans cet état, les mâchoires maintiennent l'implant replié sur lui-même, même après l'action de l'opérateur. La zone de pliage de l'implant est en saillie au-dessus des mâchoires si bien qu'il est possible de prendre en charge l'implant replié au moyen d'une pince adaptée, notamment à son introduction dans l'oeil du patient.

Dans un mode de réalisation, l'une des mâchoires est portée par la partie centrale d'une traverse appartenant à un étrier, l'autre mâchoire s'étendant entre les montants de cet étrier, l'extrémité des montants opposée à celle voisine de la traverse étant équipée du poussoir correspondant. Ainsi les montants de l'étrier peuvent constituer un moyen de centrage de l'implant entre les mâchoires lorsque celles-ci sont dans l'état ouvert de la pince.

D'autres caractéristiques et avantages ressortiront de la description d'un mode de réalisation de l'invention donné ci-après.

Il sera fait référence aux dessins annexés parmi lesquels :
- la figure 1 illustre, par une vue en plan, le dispositif selon l'invention dans son état ouvert apte à accueillir un implant,
- la figure 2 est une vue en coupe selon le plan II-II de la figure 1,
- la figure 3 est une vue en plan du dispositif dans son état refermé en l'absence d'implant,
- la figure 4 est une vue en coupe selon la ligne IV-IV de la figure 3,
- la figure 5 est une vue partielle du dispositif conforme à la figure 1 avec un implant prêt à être plié sur lui-même,
- la figure 6 est une coupe selon le plan VI-VI de la figure 5,
- la figure 7 illustre par une vue partielle le dispositif de l'invention maintenant un implant replié entre ses mâchoires,
- la figure 8 est une vue en coupe selon VIII-VIII de la figure 7.

Le dispositif représenté aux figures comporte une première mâchoire 1 portée par l'une des extrémités d'un doigt 2, l'autre extrémité de ce doigt 2 étant équipée d'un poussoir 3. Ce poussoir est en forme de barreau sensiblement perpendiculaire au doigt 2, et possède une surface extérieure concave 3a.

La mâchoire 1 possède une surface active 4 délimitée par les côtés 4a et 4b d'un dièdre rentrant faisant face au poussoir 3. Le côté 4b définit une surface sensiblement parallèle à l'axe longitudinal du doigt 2. La surface active 4 est tournée vers le poussoir 3.

Le dispositif comporte une seconde mâchoire 5 présentant comme la mâchoire 1 une surface active 6 délimitée par les côtés 6a et 6b d'un dièdre rentrant qui fait face au dièdre rentrant de la mâchoire 1. Cette mâchoire 5 est portée par la partie médiane de la traverse 7 d'un étrier dont les montants 8 et 9 encadrent le doigt 2 portant la mâchoire 1. La traverse 7 possède en-dessous de la mâchoire 5, des rebords inférieurs 10 et 11 qui délimitent une coulisse de guidage pour le doigt 2 de même que les montants 8 et 9 de l'étrier qui l'encadrent. L'extrémité des montants 8 et 9 opposée à celle portant la traverse 7 est équipée d'un poussoir 12 en forme de barreau à l'instar du poussoir 3 et comportant une surface extérieure concave 12a. La surface active 6 de la mâchoire 5 est tournée vers le poussoir 12.

Les surfaces 4b et 6b des mâchoires 1 et 5 appartiennent à un plan commun parallèle au plan de coulissement relatif du doigt et de l'étrier qui définit le plan de soutien de l'implant à plier. Entre le poussoir 12 et la traverse 7, chacun des montants 8 et 9 possède deux bossages supérieurs 13 et 14 qui sont légèrement en saillie au-dessus du plan qui contient les côtés 4b et 6b des surfaces actives 4 et 6 des mâchoires.

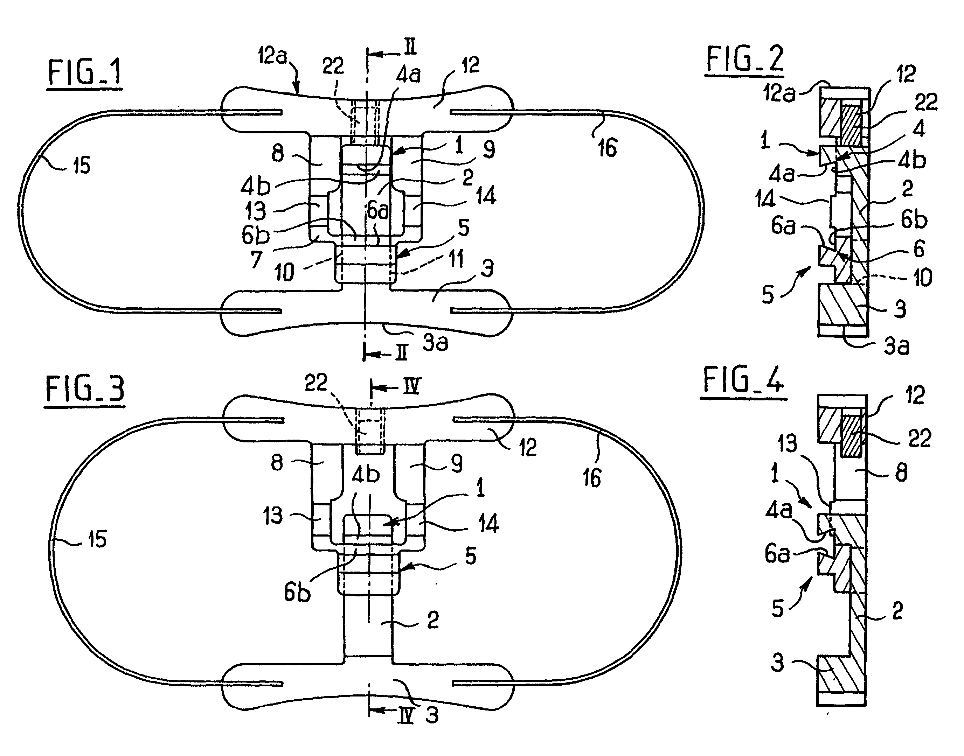

Deux lames de ressort 15 et 16 implantées chacune par leurs extrémités à l'une des extrémités de chaque poussoir 3 et 12, ont pour fonction d'une part, de maintenir les deux parties du dispositif portant les mâchoires 1 et 5 en emboîtement coulissant l'une dans l'autre et d'autre part, d'écarter élastiquement des poussoirs l'un de l'autre pour rappeler constamment les mâchoires 1 et 5 en appui l'une contre l'autre dans la position représentée à la figure 3. Dans cette position ces mâchoires butent l'une contre l'autre par le bord qui délimite chacun des côtés 4b et 6b de leur surface active, comme illustré par la figure 4.

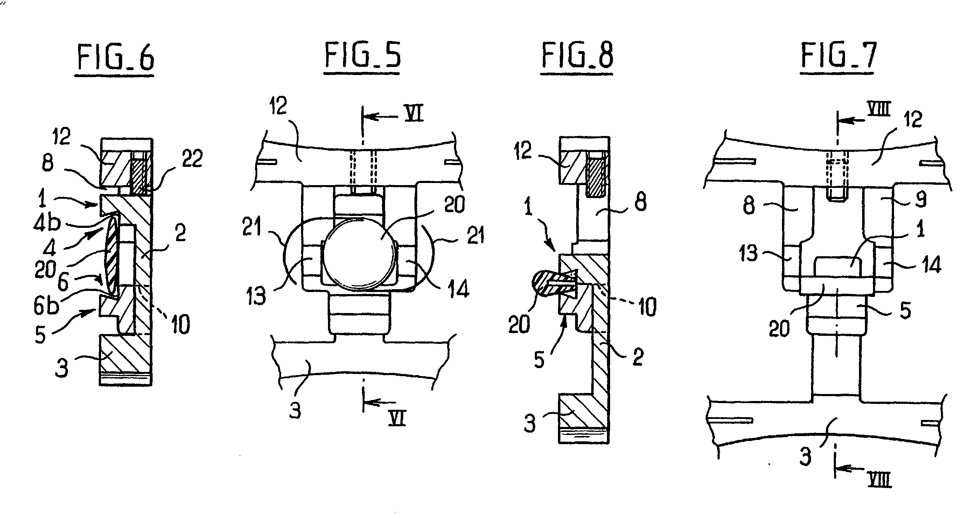

On comprend de ces figures que lorsque l'opérateur pince entre le pouce et l'index d'une main les poussoirs 3 et 12, il les rapproche et donc il écarte les mâchoires 1 et 5. On peut alors disposer entre ces mâchoires un implant 20 comme illustré par les figures 5 et 6 qui repose partiellement sur les côtés 4b et 6b de ces mâchoires ainsi que sur une partie de la surface supérieure des montants 8 et 9 située au même niveau et qui est maintenu latéralement entre les mâchoires par les bossages 13 et 14. Cet implant intraoculaire peut comporter de manière connue des anses 21 flexibles, ces anses pouvant être disposées comme illustré par la figure 5 ou dans une position perpendiculaire à celle illustrée, passant ainsi chacune d'un côté d'une mâchoire. Lorsque l'opérateur relâche le pincement des poussoirs, sous l'effet des lames de ressort 15 et 16 les mâchoires se rapprochent l'une de l'autre en déformant l'implant 20 comme illustré par les figures 7 et 8. L'implant est donc replié sur lui-même, le pli faisant saillie à l'extérieur des mâchoires pour former une partie de préhension de l'implant plié au moyen d'une pince adaptée, et est maintenu plié sous l'effet de la force de rappel des lames de ressort 15 et 16, ce qui permet à l'opérateur de lâcher le dispositif qui constitue un support d'implant plié.

Lorsqu'il s'agit d'introduire cet implant dans l'oeil, au moyen d'une pince appropriée, on saisit la partie de l'implant 20 en saillie au-dessus des mâchoires 1 et 5 et on desserre les mâchoires par pression sur les poussoirs 3 et 12 pour extraire l'implant maintenu plié par la pince qui l'a pris en charge.

On notera enfin sur les figures la présence d'une butée 22 qui limite la course d'écartement des mâchoires. Cette butée, par exemple une vis, peut être réglable ce qui permet d'ajuster l'écartement à la dimension de l'implant.

Le dispositif selon l'invention peut être réalisé en plusieurs matériaux. Réutilisable, il sera réalisé de préférence en alliage de titane, acier inoxydable ou alliage analogue connu dans le domaine de l'instrumentation chirurgicale qui permet de lui appliquer des opérations de stérilisation. A usage unique, il peut être réalisé en matière plastique injectée au moins pour les deux ensembles poussoir-mâchoire, ces pièces injectées possédant aux extrémités de chaque poussoir des logements d'encastrement de lames de ressort pouvant être également en matière plastique ou en métal.

## Revendications

1. Dispositif de pliage et de maintien plié d'un implant (20) intraoculaire souple, comportant deux mâchoires (1, 5), chaque mâchoire ayant une surface active (4, 6) en forme de dièdre rentrant et étant montée coulissante par rapport à l'autre mâchoire le long d'une direction parallèle à l'un des côtés (4b, 6b) du dièdre rentrant entre une première position où elles sont en contact l'une contre l'autre par l'extrémité du côté (4b, 6b) parallèle au coulissement et au moins une seconde position dans laquelle elles sont éloignées l'une de l'autre, **caractérisé en ce qu'**il comporte au moins un organe élastique (15, 16) attelé entre les mâchoires et dont l'effet tend à les maintenir au contact l'une de l'autre.

2. Dispositif selon la revendication 1, **caractérisé en ce que** chaque mâchoire (1, 5) est solidaire d'un poussoir (3, 12) situé en regard de sa surface active (4, 6) et au-delà de l'autre mâchoire (5, 1).

3. Dispositif selon la revendication 2, **caractérisé en ce que** l'une (5) des mâchoires est portée par la partie centrale d'une traverse (7) appartenant à un étrier, l'autre mâchoire (1) s'étendant entre les montants (8, 9) de cet étrier, l'extrémité des montants (8, 9) opposée à celle qui porte la traverse (7) étant équipée du poussoir (12) correspondant.

4. Dispositif selon la revendication 3, **caractérisé en ce que** les montants (8, 9) de l'étrier comportent un moyen (13, 14) de maintien latéral de l'implant (20) par rapport aux mâchoires (1, 5) lorsque celles-ci sont maintenues écartées dans leur seconde position.

5. Dispositif selon la revendication 3 ou la revendication 4, **caractérisé en ce que** l'autre mâchoire (1) est portée par l'une des extrémités d'un doigt (2) monté à coulissement par rapport à l'étrier sous la mâchoire (5) correspondante, l'autre extrémité du doigt (2) étant pourvue du poussoir (3) correspondant.

6. Dispositif selon l'une des revendications 3 à 5, **caractérisé en ce que** chaque poussoir (3, 12) est constitué par un barreau transversal, respectivement au doigt (2) et à l'étrier, dont la face extérieure (3a, 12a) est concave.

7. Dispositif selon la revendication 6, **caractérisé en ce que** l'organe élastique est constitué par deux lames (15, 16) de ressort, chacune formant une épingle de liaison élastique des barreaux poussoirs (3, 12).

8. Dispositif selon l'une des revendications précédentes, **caractérisé en ce qu'**il comporte une butée (22) limitant l'écartement des mâchoires (1, 5) à une valeur maximale.

## Patentansprüche

1. Vorrichtung zum Falten und Halten eines weichen intraokulären implantate (20) im gefalteten Zustand, umfassend zwei Klemmbacken (1, 5), von denen jede eine aktive Fläche (4, 6) in Form einer V-förmigen Winkelfläche hat und derart angeordnet ist, daß sie relativ zu der anderen Klemmbacke entlang einer zu einer der Seiten (4b, 6b) der V-förmigen Winkelfläche parallelen Richtung gleiten kann von einer ersten Stellung, in der die beiden Klemmbacken sich an dem Ende der Seite (4b, 6b), die parallel zur Gleitrichtung verläuft, berühren, in zumindest eine zweite Stellung, in der sie voneinander beabstandet sind, **dadurch gekennzeichnet, daß** die Vorrichtung zumindest ein elastisches Element (15, 16) umfaßt, das zwischen die Klemmbacken gespannt ist und die Tendenz hat, die beiden Klemmbacken in Kontakt miteinander zu halten.

2. Vorrichtung nach Anspruch 1, **dadurch gekennzeichnet, daß** jede Klemmbacke (1, 5) einstückig mit einem Drücker (3, 12) ausgebildet ist, der sich gegenüber der aktiven Fläche (4, 6) der Klemmbacke und jenseits der anderen Klemmbacke (5, 1) erstreckt.

3. Vorrichtung nach Anspruch 2, **dadurch gekennzeichnet, daß** die eine (5) der Klemmbacken von dem zentralen Abschnitt eines einen Teil eines gabelförmigen Bügels bildenden Querelements (7) getragen ist, während sich die andere Klemmbacke (1) zwischen den Schenkeln (8, 9) dieses Bügels erstreckt, wobei das Ende der Schenkel (8, 9), das dem das Querelement (7) tragenden Ende entgegengesetzt ist, mit dem entsprechenden Drücker (12) ausgestattet ist.

4. Vorrichtung nach Anspruch 3, **dadurch gekennzeichnet, daß** die Schenkel (8, 9) des gabelförmigen Bügels eine Vorrichtung (13, 14) umfassen zum seitlichen Halten des Implantats (20) relativ zu den Klemmbacken (1, 5), wenn diese in ihrer zweiten Stellung voneinander beabstandet gehalten sind.

5. Vorrichtung nach Anspruch 3 oder 4, **dadurch gekennzeichnet, daß** die andere Klemmbacke (1) von einem der Enden eines Stifts (2) gehalten ist, der relativ zu dem dreiseitigen Bügel unter der entsprechenden Klemmmbacke (5) gleitend angeordnet ist, während das andere Ende des Stifts (2) mit dem entsprechenden Drücker (3) ausgestattet ist.

6. Vorrichtung nach einem der Ansprüche 3 bis 5, **dadurch gekennzeichnet, daß** jeder Drücker (3, 12) von einem Querstab gebildet ist, der relativ zu dem Stift, bzw. dem dreiseitigen Bügel quer verläuft und dessen Außenfläche (3a, 12a) konkav ist.

7. Vorrichtung nach Anspruch 6, **dadurch gekennzeichnet, daß** das elastische Element von zwei Federblättern (15, 16) gebildet ist, die je einen elastischen Bügel zum elastischen Verbinden der Drücker (3, 12) bilden.

8. Vorrichtung nach einem der vorhergehenden Ansprüche, **dadurch gekennzeichnet, daß** ie einen Anschlag (22) umfaßt, der die Beabstandung der Klemmbacken (1, 5) voneinander auf einen Maximalwert begrenzt.

## Claims

1. A device for folding a flexible intraocular implant (20) and for keeping it folded, the device comprising two jaws (1, 5), each jaw having an active surface (4, 6) forming a reentrant dihedral, and each being slidably mounted relative to the other jaw along a direction parallel to one of the faces (4b, 6b) of the reentrant dihedral, between a first position in which the jaws are in contact against each other via the ends of the faces (4b, 6b) parallel to the sliding direction, and at least one second position in which the jaws are moved apart from each other, the device being **characterized in that** it includes at least one resilient member (15, 16) coupled between the jaws and having the effect of tending to keep them in contact with each other.

2. A device according to claim 1, **characterized in that** each jaw (1, 5) is secured to a pushbutton (3, 12) situated facing its active surface (4, 6) and beyond the other jaw (5, 1).

3. A device according to claim 2, **characterized in that** one of the jaws (5) is carried by the central portion of a cross-member (7) belonging to a stirrup piece, with the other jaw (1) extending between the uprights (8, 9) of the stirrup piece, the ends of the uprights (8, 9) remote from their ends carrying the cross-member (7) being fitted with the corresponding pushbutton (12).

4. A device according to claim 3, **characterized in that** the uprights (8, 9) of the stirrup piece include means (13, 14) for holding the sides of the implant (20) relative to the jaws (1, 5) when the jaws are held apart in their second position.

5. A device according to claim 3 or 4, **characterized in that** the other jaw (1) is carried by one of the ends of a finger (2) slidably mounted relative to the stirrup piece behind the corresponding jaw (5), the other end of the finger (2) being provided with the corresponding pushbutton (3).

6. A device according to any one of claims 3 to 5, **characterized in that** each pushbutton (3, 12) is constituted by a bar extending transversely respectively relative to the finger (2) and to the stirrup piece, with the outside face (3a, 12a) of each bar being concave.

7. A device according to claim 6, **characterized in that** the resilient member is constituted by two spring blades (15, 16) each forming a spring clip interconnecting the pushbutton bars (3, 12).

8. A device according to any preceding claim, **characterized in that** it includes an abutment (22) limiting the spacing of the jaws (1, 5) to a maximum value.
